# EUROPÄISCHE PATENTANMELDUNG

(11) **EP 4 015 678 A1**
(43) Veröffentlichungstag der Anmeldung: **22.06.2022**
(21) Anmeldenummer: 20214821.9
(22) Anmeldetag: 17.12.2020
(51) Int. Cl.: D01D 5/00

(54) **VORRICHTUNG UND VERFAHREN ZUR HERSTELLUNG EINES ANISOTROPEN FASERGERÜSTS DURCH ELEKTROSPINNING**

(71) Anmelder: Medizinische Universität Wien, 1090 Wien (AT); Ludwig Boltzmann Gesellschaft - österreichische Vereinigung zur Förderung der wissenschaftlichen Forschung, 1090 Wien (AT)
(72) Erfinder: Christian, Grasl, 1210 Wien (AT); Schima, Heinrich, 1020 Wien (AT); Bergmeister, Helga, 1030 Wien (AT)
(74) Vertreter: Puchberger & Partner Patentanwälte

(57) **Zusammenfassung**

Vorrichtung und Verfahren zur Herstellung eines anisotropen Fasergerüsts (10), insbesondere einer Gefäßprothese durch Elektrospinning, umfassend ein Reservoir (1), eine Spinndüse (2) zur Abgabe eines Materialstrahls (8) und eine relativ zur Spinndüse (2) rotierbare, elektrisch leitende Abscheidespindel (3), wobei zumindest eine vorzugsweise variable, zweipolige elektrische Gleichspannungsquelle (4, 4a, 4b) zur Bereitstellung einer elektrischen Potentialdifferenz zwischen der Spinndüse (2) und der Abscheidespindel (3) vorgesehen ist, und wobei ein die Abscheidespindel (3) und die Spinndüse (2) zumindest teilweise umschließendes, vorzugsweise geerdetes Gehäuse (7) vorgesehen ist, wobei eine gesteuerte Schalteinheit (5) vorgesehen ist, die dazu ausgebildet ist, während des Elektrospinnens die Abscheidespindel (3) zumindest einmal, vorzugsweise mehrmals von der Gleichspannungsquelle (4, 4a, 4b) zu trennen.

## Beschreibung

Die Erfindung betrifft eine Vorrichtung und ein Verfahren zur Herstellung eines röhrenförmigen Fasergerüsts durch Elektrospinning.

Elektrospinning ist eine Technologie zur Herstellung von Fasern aus natürlichen und synthetischen Polymeren mit Durchmessern von ungefähr 30 nm bis 3 µm. Die Faserbildung erfolgt dabei durch die Beschleunigung der im Polymer induzierten Ladungsträger in einem starken elektrischen Feld, was letztlich zur Verstreckung des viskoelastischen Materials führt. Das elektrische Feld wird im einfachsten Fall durch Anlegen einer Hochspannung zwischen einem Reservoir, welches die Polymerlösung enthält und einer Abscheidespindel, auf der die Fasern deponiert werden, erzeugt.

Das Reservoir ist mit einer Spinndüse verbinden, durch welche der Polymerlösungsstrahl austritt. Durch die Wahl der Spinnparameter (z.B. Abstand zwischen Reservoir und Abscheidespindel, Viskosität, Oberflächenspannung und Leitfähigkeit der Polymerlösung, gewählte Hochspannung, ...) können die Eigenschaften der gebildeten Fasern (Durchmesser, Faserabstand, Homogenität,...) beeinflusst werden.

Insbesondere die Herstellung von medizinischen Gefäßprothesen erfordert die Erzeugung anisotroper, meist röhrenförmiger Fasergerüste (sog. Grafts oder Stentgrafts). Um durch Elektrospinning röhrenförmige Fasergerüste zu erzeugen, ist die Verwendung einer rotierbaren Abscheidespindel bekannt, auf welche der Materialstrahl abgeschieden wird. Die Abscheidespindel ist länglich und stabförmig und wird auch als Mandrel bezeichnet.

Zur Bereitstellung einer elektrischen Potentialdifferenz zwischen der Spinndüse und der Abscheidespindel ist die Spinndüse mit dem ersten elektrischen Pol einer zweipoligen elektrischen Gleichspannungsquelle verbunden. Dabei kann es sich insbesondere um einen variabel einstellbaren Hochspannungsgenerator handeln. Die Polymerlösung kann mit einer positiven oder negativen Hochspannung beaufschlagt werden, wobei zwischen Reservoir bzw. Spinndüse und Abscheidespindel eine Potentialdifferenz erzeugt wird.

Es hat sich gezeigt, dass die Bioverträglichkeit der hergestellten Grafts und insbesondere das Einwachsen körpereigener Zellen in die Grafts von der lokalen Strukturierung des hergestellten Fasergerüsts abhängig ist. Folglich besteht eine der Aufgaben der Erfindung darin, ein möglichst einfaches und robustes Verfahren sowie eine entsprechende Vorrichtung zu schaffen, die eine lokale Strukturierung des hergestellten Fasergerüsts ermöglicht.

Erfindungsgemäß wird diese Aufgabe durch eine Vorrichtung gemäß Anspruch 1 und ein Verfahren gemäß Anspruch 8 gelöst.

Eine erfindungsgemäße Vorrichtung ist zur Herstellung eines anisotropen Fasergerüsts, insbesondere einer Gefäßprothese durch Elektrospinning ausgebildet und umfasst ein Reservoir, eine Spinndüse zur Abgabe eines Materialstrahls und eine relativ zur Spinndüse rotierbare, elektrisch leitende Abscheidespindel. Es ist zumindest eine elektrische Gleichspannungsquelle zur Bereitstellung einer elektrischen Potentialdifferenz zwischen der Spinndüse und der Abscheidespindel vorgesehen. Es kann sich dabei insbesondere um eine variable zweipolige Hochspannungsquelle handeln.

Ferner ist ein, die Abscheidespindel und die Spinndüse zumindest teilweise umschließendes Gehäuse vorgesehen, wobei das Gehäuse vorzugsweise geerdet ist. Es ist eine gesteuerte Schalteinheit vorgesehen, die dazu ausgebildet ist, während des Elektrospinnens die Abscheidespindel abwechselnd mit der Gleichspannungsquelle zu verbinden, also auf ein definiertes Potential zu legen, und wieder von dieser zu trennen, also die Abscheidespindel floatend zu lassen. Die Abscheidespindel kann aus einem elektrisch leitenden Material bestehen oder dieses umfassen, beispielsweise Stahl, Aluminium oder Kupfer. Die Abscheidespindel kann auch als Abscheideelektrode bezeichnet werden.

Es können insbesondere zwei Ausführungsformen erfindungsgemäß vorgesehen sein:
In einer **ersten Ausführungsform** ist die Spinndüse mit einem ersten elektrischen Pol (z.B. dem Plus-Pol) der Gleichspannungsquelle verbunden und die Abscheidespindel mit einem zweiten elektrischen Pol (z. B. dem Minus-Pol) der Gleichspannungsquelle verbunden. Die Schalteinheit ist dazu ausgebildet, während des Elektrospinnens den zweiten elektrischen Pol der Gleichspannungsquelle zumindest einmal, vorzugsweise mehrmals mit der Abscheidespindel zu verbinden und nach einer bestimmten Zeitdauer wieder von der Abscheidespindel zu trennen. Insbesondere wird dadurch die Abscheidespindel zwischen dem Ground-Potential (0 V) und einem undefinierten Potential (floatend) geschaltet. Diese Ausführungsform kann dann vorgesehen sein, wenn die von der Gleichspannungsquelle generierbare Potentialdifferenz (z.B. 15 kV) für den Spinnvorgang ausreichend ist; während des Spinnvorgangs liegt die Spinndüse auf +15 kV und die Abscheidespindel auf 0 V bzw. floatend.

In einer **zweiten Ausführungsform** sind zwei Gleichspannungsquellen vorgesehen, wobei die Spinndüse mit einem ersten elektrischen Pol (z. B. dem Plus-Pol) der ersten Gleichspannungsquelle verbunden ist, und die Abscheidespindel mit einem ersten elektrischen Pol (z. B. dem Plus-Pol) der zweiten Gleichspannungsquelle verbunden ist. Die Schalteinheit ist dazu ausgebildet, während des Elektrospinnens den ersten elektrischen Pol der zweiten Gleichspannungsquelle zumindest einmal, vorzugsweise mehrmals, mit der Abscheidespindel zu verbinden und nach einer bestimmten Zeitdauer wieder von der Abscheidespindel zu trennen.

Diese zweite Ausführungsform kann insbesondere dann vorgesehen sein, wenn die von der Gleichspannungsquelle generierbare Potentialdifferenz (z.B. 15 kV) für den Spinnvorgang nicht ausreichend ist; es werden dann zwei Gleichspannungsquellen eingesetzt. Während des Spinnvorgangs liegt die Spinndüse auf +15 kV und die Abscheidespindel auf einem Potentialwert von unter 0 V, beispielsweise - 2 kV, bzw. floatend, sodass eine Potentialdifferenz von 17 kV erzeugt wird. Die zweiten elektrischen Pole der Gleichspannungsquellen (die Minus-Pole) sind dabei elektrisch verbunden.

Durch das erfindungsgemäße Umschalten zwischen 0 V (bzw. einem negativen Potential) und einem undefinierten (floatenden) Potential während des Elektrospinnens wird erreicht, dass sich die strukturellen Eigenschaften des erzeugten Fasergerüsts während des Spinnvorganges ändern. Dadurch können die Dicke, die Beschaffenheit und die mechanischen Eigenschaften des erzeugten Fasergerüsts auf einfachem Wege, rein elektrisch und ohne mechanischen Eingriff, verändert werden.

Beispielsweise hat sich gezeigt, dass manche Materialien beim Elektrospinnen zur Wellen- bzw. Lappenbildung neigen, wenn die Abscheidespindel auf GND-Potential liegt, d.h. der äußere Umfang des Fasergerüst wird unregelmäßig, das Fasergerüst erstreckt sich in radialer Richtung inhomogen nach außen. Durch das erfindungsgemäße Umschalten des zweiten elektrischen Pols der Gleichspannungsquelle nach einer bestimmten Spinndauer hat sich überraschenderweise gezeigt, dass dies die Wellen- bzw. Lappenbildung am äußeren Umfang unterdrückt, da sie sich an das ursprüngliche zylindrische Fasergerüst anlegen, wobei sich Hohlräume im Fasergerüst bilden. Es entstehen bei geeignet gewählten Zeitdauern Hohlräume mit einem typischen Durchmesser von über 5 µm.

Durch Wahl der Zeitdauer zwischen dem Umschalten kann die Größe der durch die Lappen gebildeten Hohlräume im Fasergerüst variiert werden. Insbesondere hat sich gezeigt, dass die Lappen und somit auch die Hohlräume umso größer sind, je länger der zweite Pol mit der Abscheidespindel verbunden ist.

Mit anderen Worten, wenn die Abscheidespindel auf einem definierten Potential liegt (insbesondere GND oder ein Potential kleiner 0 V), dann bilden sich Unregelmäßigkeiten im Fasergerüst, die geschlossen werden, wenn die Abscheidespindel von dem definierten Potential getrennt wird und floatet. Durch dieses Schließen der Unregelmäßigkeiten entstehen also Hohlräume, deren Ausdehnung durch das Umschalten der Spannungsverhältnisse beeinflussbar ist.

Da die gebildeten Wellen bzw. Lappen unterschiedliche makroskopische Eigenschaften als das Grundgerüst aufweisen, beispielsweise unterschiedliche Festigkeit und Elastizität, können im Fasergerüst gezielt Zwischenschichten mit den gewünschten Eigenschaften erzeugt werden. Sowohl die so hergestellten Hohlräume, als auch die Zwischenschichten im Fasergerüst bieten Vorteile bei der Verwendung der gebildeten Fasergerüste als Gefäßprothesen, beispielsweise hinsichtlich ihrer Biokompatibilität bzw. Anpassung an die Flexibilität der behandelten Gefäße.

Erfindungsgemäß kann eine, bezüglich des ausgestoßenen Materialstrahls hinter der Abscheidespindel angeordnete Rückelektrode vorgesehen sein. Eine derartige Rückelektrode kann dazu dienen, den Materialstrahl zu stabilisieren, wenn die Abscheidespindel auf einem undefinierten Potential liegt, also floatend ist. Zu diesem Zweck ist die Schalteinheit dazu ausgebildet, während des Elektrospinnens den zweiten elektrischen Pol der Gleichspannungsquelle, bzw. den ersten elektrischen Pol der zweiten Gleichspannungsquelle mit der Rückelektrode zu verbinden, wenn die Abscheidespindel floatend ist.

Erfindungsgemäß ist vorgesehen, dass ein die Abscheidespindel und die Spinndüse zumindest teilweise umschließendes Gehäuse vorgesehen ist. Das Gehäuse kann mit dem zweiten elektrischen Pol (Minus-Pol bzw. GND) der Gleichspannungsquelle bzw. mit den zweiten elektrischen Polen (Minus-Pole bzw. GND) der Gleichspannungsquellen verbunden sein. Insbesondere kann das Gehäuse geerdet sein. Dadurch wird sichergestellt, dass das Gehäuse der Vorrichtung geerdet ist, also auf einem Potential von 0 V liegt und somit einen "Faradayschen Käfig" bildet.

Die somit erzeugte Potentialdifferenz kann etwa 5 kV bis 20 kV, vorzugsweise 8 kV bis 17 kV, besonders bevorzugt 10 kV bis 15 kV betragen, wobei das Potential an der Abscheidespindel vorzugsweise kleiner ist als das Potential an der Spinndüse, insbesondere kleiner oder gleich 0 V, beispielsweise - 2kV.

Die Umschaltung der Gleichspannungsquelle(n) kann manuell oder durch eine elektronische Steuereinheit erfolgen. Eine derartige elektronische Steuereinheit kann insbesondere zur Ansteuerung zumindest der Gleichspannungsquelle(n) und der Schalteinheit vorgesehen sein. Die Steuereinheit kann aber auch andere Elemente der erfindungsgemäßen Vorrichtung steuern, beispielsweise ein Ventil des Reservoirs zur Steuerung der Abgabe des Materials, oder Motoren zur Drehung und/oder Verschiebung der Abscheidespindel.

Die Abscheidespindel kann als relativ zur Spinndüse rotierbare Spindel ausgebildet sein, die in einer im Wesentlichen orthogonal zum ausgestoßenen Materialstrahl verlaufenden Achse rotierbar und/oder entlang dieser Achse verschiebbar ist. Insbesondere kann vorgesehen sein, dass die Abscheidespindel entlang dieser Achse in einem sinusförmigen oder dreieckförmigen Schwingungsverlauf hin- und her-bewegbar ist, um ein längliches Fasergerüst zu erzeugen.

Ein sinusförmiger Schwingungsverlauf der Verschiebung resultiert in erweiterten Endbereichen des Fasergerüsts, die in weiterer Folge als Stützringe der Gefäßprothese dienen können. Alternativ oder zusätzlich kann vorgesehen sein, dass die Rückelektrode entlang dieser Achse in einem sinusförmigen oder dreieckförmigen Schwingungsverlauf hin- und her-bewegbar ist, um die längliche Erstreckung des Fasergerüsts zu erzeugen. Darüber hinaus kann durch Verlangsamung oder Verringerung der Oszillation der Verschiebung an vorgegebenen Stellen eine lokale Verdickung der Gefäßwandstärke erreicht werden, um durch die dadurch entstehenden Stützringe ein Kollabieren oder eine externe Kompression des Gefäßes im Patienten zu verhindern. Die Abscheidespindel kann auch in Richtung des Materialstrahls verschiebbar sein, um den Abstand zwischen Spinndüse und Abscheidespindel einstellen zu können.

Die Abscheidespindel kann einen Durchmesser von etwa 2 mm und eine Drehzahl während des Elektrospinnens von etwa 250 Umdrehungen pro Minute aufweisen. Der Abstand zwischen der Spinndüse und der Abscheidespindel kann etwa 5 cm bis 12 cm, beispielsweise etwa 8 cm betragen. Der Durchmesser der Abscheidespindel kann auch deutlich größer sein, beispielsweise 5 mm, 10 mm, 20 mm, 30 mm, oder 36 mm, etwa wenn die Gefäßprothese in die Aorta eines Erwachsenen eingesetzt werden soll. Insbesondere kann eine Durchmesser der Abscheidespindel von etwa 2 mm für die Erstellung von Gefäßprothesen zum Ersatz kleinerer Gefäße (Arterien, aber auch Venen, Lymphgefäße oder Liquor-Abflußsysteme) vorgesehen sein. Alternativ kann ein Durchmesser von etwa 4 mm bis etwa 8 mm für mittlere Gefäße und Dialyse-Shunts, aber größere Durchmesser bis hin zum Ersatz von Abschnitten der Aorta, der Pulmonalarterie oder der großen Venen vorgesehen sein.

Bei Wahl eines größeren Durchmessers der Abscheidespindel wird vorzugsweise auch die Drehzahl entsprechend reduziert, um eine im Wesentlichen gleiche Umfangsgeschwindigkeit zu erzielen. Beispielsweise kann bei einem Durchmesser der Abscheidespindel von etwa 5 mm eine Drehzahl von etwa 80 - 120 Umdrehungen pro Minute vorgesehen sein. Bei großen Durchmessern können auch mehrere Spinndüsen vorgesehen sein.

Im Bereich zwischen der Spinndüse und der Abscheidespindel können ferner Stabilisierungselektroden vorgesehen sein, die vorzugsweise plattenförmig sind und vorzugsweise mit dem ersten elektrischen Pol oder mit dem zweiten elektrischen Pol der Gleichspannungsquelle(n) verbunden sind. Derartige Stabilisierungselektroden können dazu dienen, den ausgestoßenen Materialstrahl zu stabilisieren und gegebenenfalls zu fokussieren.

Bei dem ausgestoßenen Material kann es sich um ein Gemisch aus einem Polymer und einem Lösungsmittel handeln. Beispielsweise kann der Materialstrahl eine Lösung aus 5 Gew.-% Polydioxanon (PDS) in Hexafluoro2propanol, oder eine Lösung aus 5 Gew.-% thermoplastisches Polyurethan (TPU) in Hexafluoro2propanol umfassen.

Die Erfindung betrifft ferner ein Verfahren zur Herstellung eines anisotropen Fasergerüsts, insbesondere einer Gefäßprothese durch Elektrospinning, wobei ein Materialstrahl aus einem Reservoir über eine Spinndüse auf eine rotierende, elektrisch leitende Abscheidespindel geführt wird, und wobei eine elektrische Potentialdifferenz zwischen der Spinndüse und der Abscheidespindel gebildet wird, indem die Spinndüse und die Abscheidespindel mit einer vorzugsweise variablen, zweipoligen elektrischen Gleichspannungsquelle verbunden werden.

Erfindungsgemäß ist eine gesteuerte Schalteinheit vorgesehen, welche die Abscheidespindel während des Spinnvorgangs zumindest einmal, vorzugsweise mehrmals mit der Gleichspannungsquelle verbindet und nach Ablauf einer vorbestimmten Zeitdauer wieder von dieser trennt. Durch diese Veränderung der elektrischen Potentialdifferenz zwischen der Abscheidespindel und der Spinndüse während des Elektrospinnens wird eine lokale Strukturierung des abgeschiedenen Fasergerüsts erzielt.

In einer ersten Ausführungsform wird die Potentialdifferenz gebildet, indem die Spinndüse mit einem ersten elektrischen Pol der Gleichspannungsquelle verbunden wird, und die Abscheidespindel mit einem zweiten elektrischen Pol der Gleichspannungsquelle verbunden wird. Die Schalteinheit verbindet den zweiten elektrischen Pol (Minus-Pol, GND) der Gleichspannungsquelle mit der Abscheidespindel und trennt ihn nach einer bestimmten Zeitdauer wieder von dieser. Durch die einmalige oder mehrfache Veränderung der elektrischen Potentialdifferenz zwischen der Abscheidespindel und der Spinndüse während des Elektrospinnens kann eine lokale Strukturierung des abgeschiedenen Fasergerüsts erzielt werden.

In einer alternativen Ausführungsform des erfindungsgemäßen Verfahrens wird die Potentialdifferenz gebildet, indem die Spinndüse mit einem ersten elektrischen Pol einer ersten Gleichspannungsquelle verbunden und die Abscheidespindel mit einem ersten elektrischen Pol einer zweiten Gleichspannungsquelle verbunden.

Die Schalteinheit verbindet während des Elektrospinnens den ersten elektrischen Pol der zweiten Gleichspannungsquelle zumindest einmal, vorzugsweise mehrmals mit der Abscheidespindel und trennt ihn nach Ablauf einer bestimmten Zeitdauer wieder von dieser.

In beiden Ausführungsformen kann vorgesehen sein, dass die Schalteinheit den zweiten elektrischen Pol der Gleichspannungsquelle, oder den ersten elektrischen Pol der zweiten Gleichspannungsquelle zumindest einmal, vorzugsweise mehrmals von der Abscheidespindel trennt und mit einer, bezüglich des Materialstrahls hinter der Abscheidespindel angeordneten Rückelektrode verbindet.

Die Abscheidespindel kann während des Elektrospinnens relativ zur Spinndüse entlang einer, in einer im Wesentlichen orthogonal zum ausgestoßenen Materialstrahl verlaufenden Achse rotieren und/oder entlang dieser Achse verschoben werden. Insbesondere kann die Abscheidespindel entlang dieser Achse in einem sinusförmigen oder dreieckförmigen Schwingungsverlauf hin- und her-bewegt werden. Alternativ oder zusätzlich kann auch die Rückelektrode entlang dieser Achse in einem sinusförmigen oder dreieckförmigen Schwingungsverlauf hin- und her-bewegt werden.

Die Schalteinheit kann während des Elektrospinnens für eine erste Zeitdauer t₁ die Abscheidespindel mit der Gleichspannungsquelle verbinden, und anschließend für eine zweite Zeitdauer t₂ die Abscheidespindel von der Gleichspannungsquelle trennen. Die Zeitdauern t₁, t₂ können jeweils etwa 5 Minuten bis 10 Minuten betragen. Der Vorgang kann etwa 5 mal bis etwa 10 mal wiederholt werden.

Natürlich kann der Vorgang auch in umgekehrter Reihenfolge durchgeführt werden, wobei die Schalteinheit zunächst für eine Zeitdauer t₂ die Abscheidespindel von der Gleichspannungsquelle trennt, und anschließend für eine Zeitdauer t₁ die Abscheidespindel mit der Gleichspannungsquelle verbindet.

Die Zeitdauern t₁ und t₂ können auch unterschiedlich sein. Beispielsweise kann die Zeitdauer t₁ etwa 5 bis 10 Minuten betragen, und die Zeitdauer t₂ etwa 2 bis 5 Minuten betragen.

Die Zeitdauer t₂ ist aber vorzugsweise geringer als die Zeitdauer t₁. Die gesamte Dauer des Elektrospinnens kann die Summe aus t₁ und t₂ sein. Die Schalteinheit kann dazu ausgebildet sein, diese Zeitdauern und den Umschaltzeitpunkt in Abhängigkeit der Anwendung und des gesponnenen Material unterschiedliche einzustellen, wobei entsprechende Werte beispielsweise aus einer Datenbank bezogen werden können.

Die Drehzahl der Abscheidespindel kann etwa 250 Umdrehungen pro Minute betragen, und der Durchmesser der Abscheidespindel kann etwa 2 mm, 5 mm, 10 mm, 20 mm, 30 mm, oder 36 mm betragen. Die Förderrate des Materialstrahls aus dem Reservoir kann etwa 0,5 - 2 ml/h, insbesondere etwa 0,7ml/h betragen.

Weitere Merkmale der Erfindung ergeben sich aus den Ansprüchen, der nachfolgenden Beschreibung der Ausführungsbeispiele und den Figuren. Es zeigen:
Fig. 1a eine schematische Darstellung einer ersten Ausführungsform einer erfindungsgemäßen Vorrichtung;
Fig. 1b eine schematische Darstellung einer zweiten Ausführungsform einer erfindungsgemäßen Vorrichtung;
Fig. 1c eine schematische Darstellung einer dritten Ausführungsform einer erfindungsgemäßen Vorrichtung;
Fig. 1d eine schematische Darstellung einer vierten Ausführungsform einer erfindungsgemäßen Vorrichtung;
Figs. 2a - 3b mikroskopische Aufnahmen eines Querschnitts zweier mit herkömmlichen Verfahren hergestellter Fasergerüste;
Figs. 4a - 4b mikroskopische Aufnahmen eines Querschnitts eines mit einem erfindungsgemäßen Verfahren hergestellten Fasergerüstes.

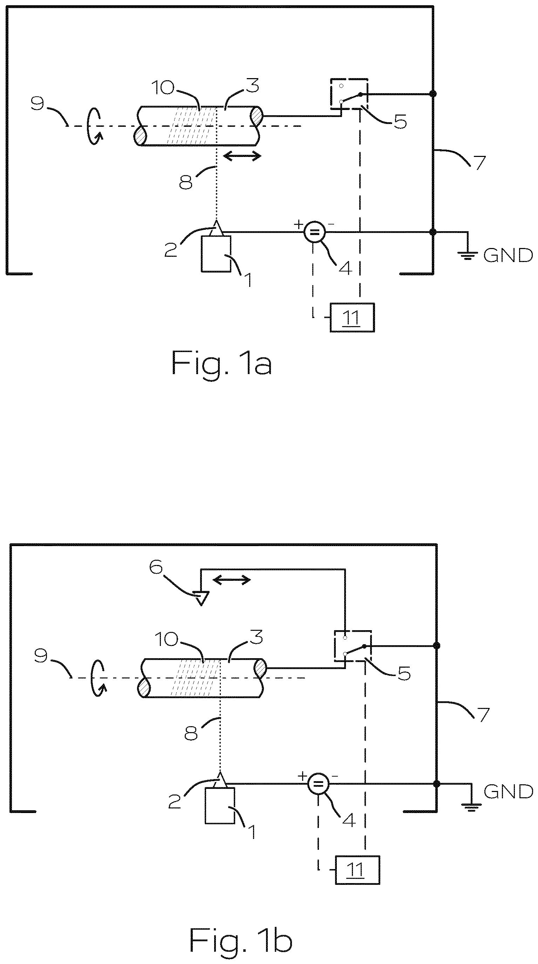

Fig. 1a zeigt eine schematische Darstellung einer ersten Ausführungsform einer erfindungsgemäßen Vorrichtung. Die Vorrichtung ist zur Herstellung eines anisotropen Fasergerüsts 10 in Form einer Gefäßprothese durch Elektrospinning ausgebildet und umfasst ein Reservoir 1, in dem eine Polymerlösung bereitgestellt wird, und eine damit verbundene Spinndüse 2 zur Abgabe eines Materialstrahls. Die Spinndüse 2 ist mit einem ersten Pol einer elektrischen Gleichspannungsquelle 4 verbunden, hier dem Plus-Pol dieser Gleichspannungsquelle 4.

Der Minus-Pol der Gleichspannungsquelle 4 ist geerdet (GND) und mit einem Gehäuse 7 verbunden. In einem Abstand von etwa 8 cm von der Spinndüse 2 ist eine rotierbare Abscheidespindel 3 vorgesehen. Die Gleichspannungsquelle 4 stellt eine Hochspannung von etwa 8 kV zur Verfügung.

Die Abscheidespindel 3 ist elektrisch leitfähig, beispielsweise aus Stahl, und über eine Schalteinheit 5 mit dem Gehäuse 7, also dem Minus-Pol der Gleichspannungsquelle 4 verbunden. Es bildet sich eine elektrische Potentialdifferenz zwischen der Spinndüse 2 und der Abscheidespindel 3, welche dazu führt, dass bei der Spinndüse 2 austretendes Material in Form eines Materialstrahls 8 auf die sich drehende Abscheidespindel 3 abgegeben wird. Durch die Drehung der Abscheidespindel 3 wickelt sich der Materialstrahl 8 auf und bildet ein schematisch dargestelltes rohrförmiges Fasergerüst 10. Durch die mit einem Doppelpfeil angedeutete periodische sinus- oder dreieckförmige Verschiebung der Abscheidespindel 3 entlang der Achse 9 kann die Länge des hergestellten Fasergerüsts 10 eingestellt werden.

Es ist eine gesteuerte elektronische Schalteinheit 5 vorgesehen, die dazu ausgebildet ist, während des Elektrospinnens den zweiten elektrischen Pol der Gleichspannungsquelle 4 mit der elektrisch leitfähigen Abscheidespindel 3 zu verbinden und wieder von dieser zu trennen. Mit anderen Worten, die Abscheidespindel alterniert zwischen einem Zustand (a), bei dem sie auf GND-Potential gehalten wird, und einem Zustand (b), bei dem sie frei floatet.
Im Zustand (b) bildet sich dennoch ein Materialstrahl 8, da sich in diesem Fall eine Potentialdifferenz zwischen der Spinndüse 2 und dem Gehäuse 7 einstellt, sodass der Materialstrahl 8 vom Gehäuse 7 angezogen wird und auf die Abscheidespindel 3 trifft.

Im Zustand (a) entsteht eine Struktur mit Verwerfungen an der Oberfläche, die als Wellen oder radial nach außen gerichtete, in Längsrichtung verlaufende Lappen beobachtet wurden. Im Zustand (b) bildet sich eine weitgehend gleichmäßige Struktur ohne größere Hohlräume.

Durch zumindest einmaliges Umschalten zwischen Zustand (a) und Zustand (b) kommt es nur im Zustand (a) zur Lappenbildung, während sich im Zustand (b) die gebildeten Lappen homogen über das bereits vorhandene Fasergerüst stülpen. Dadurch kommt es zur Bildung von kleineren oder größeren Kavitäten in der Umfangswand des Fasergerüsts.

Fig. 1b zeigt eine schematische Darstellung einer zweiten Ausführungsform einer erfindungsgemäßen Vorrichtung. Diese entspricht weitgehend der ersten Ausführungsform mit dem Unterschied, dass eine, bezüglich des ausgestoßenen Materialstrahls 8 hinter der Abscheidespindel 3 angeordnete elektrisch leitfähige Rückelektrode 6 vorgesehen ist. Die Schalteinheit 5 schaltet den zweiten elektrischen Pol (den Minus-Pol) der Gleichspannungsquelle 4 zwischen der Abscheidespindel 3 und der Rückelektrode 6 um. Im Zustand (b), wenn also die Abscheidespindel 3 floatet, bilden sich auch Fasern zwischen Rückelektrode 6 und Abscheidespindel 3, die von dieser aufgrund deren Rotation aufgewickelt werden, es bilden sich jedoch keine Lappen. Durch die mit einem Doppelpfeil angedeutete periodische sinus- oder dreieckförmige Verschiebung der Rückelektrode 6 parallel zur Achse 9 kann die Länge des hergestellten Fasergerüsts 10 eingestellt werden.

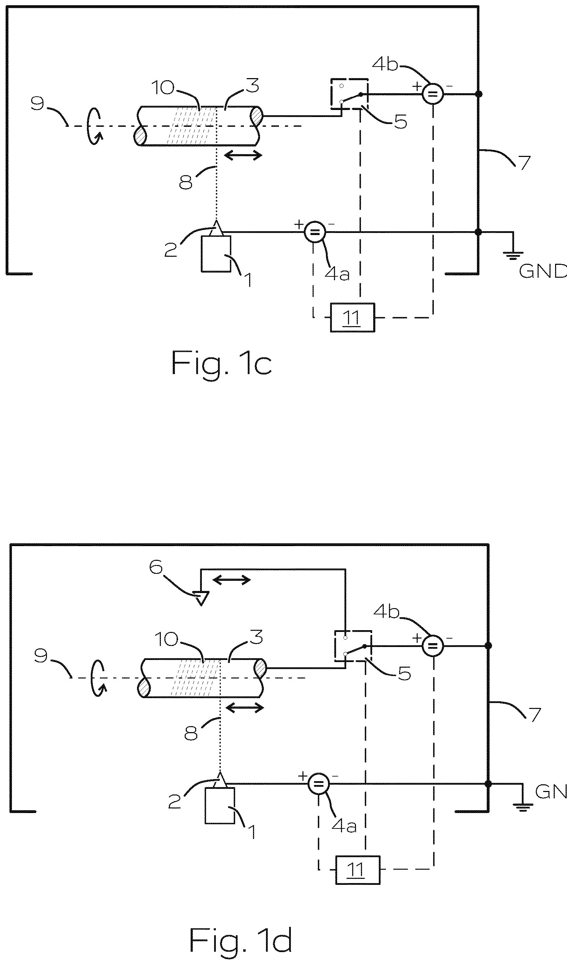

Fig. 1c und Fig. 1d zeigen schematische Darstellungen einer dritten und vierten Ausführungsform einer erfindungsgemäßen Vorrichtung. Diese Ausführungsformen entsprechen jenen aus Fig. 1a und Fig. 1b, wobei jedoch zwei Gleichspannungsquellen 4a, 4b vorgesehen sind. Dies erlaubt die Erzeugung höherer Potentialdifferenzen als mit einer einzigen Gleichspannungsquelle.

Die Spinndüse 2 ist mit dem ersten elektrischen Pol der ersten Gleichspannungsquelle 4a verbunden, und die Abscheidespindel 3 ist mit einem ersten elektrischen Pol der zweiten Gleichspannungsquelle 4b verbunden. Die Schalteinheit 5 ist dazu ausgebildet, während des Elektrospinnens den ersten elektrischen Pol der zweiten Gleichspannungsquelle 4b alternierend mit der Abscheidespindel 3 zu verbinden und wieder von dieser zu trennen.

Im Ausführungsbeispiel nach Fig. 1d ist eine, bezüglich des ausgestoßenen Materialstrahls 8 hinter der Abscheidespindel 3 angeordnete Rückelektrode 6 vorgesehen, wobei die Schalteinheit 5 dazu ausgebildet ist, während des Elektrospinnens den ersten elektrischen Pol der zweiten Gleichspannungsquelle 4b alternierend mit der Abscheidespindel 3 zu verbinden und von der Abscheidespindel 3 zu trennen und mit der Rückelektrode 6 zu verbinden. In diesem Ausführungsbeispiel ist sowohl die Abscheidespindel 3, als auch die Rückeleketrode 6 entlang der mit Doppelpfeilen angedeuteten Richtung periodische sinus- oder dreieckförmig verschiebbar, um die Länge des hergestellten Fasergerüsts 10 einzustellen.

In beiden Ausführungsbeispielen nach Fig. 1c und Fig. 1d ist ein die Abscheidespindel 3 und die Spinndüse 2 teilweise umschließendes Gehäuse 7 vorgesehen, wobei das Gehäuse mit den zweiten elektrischen Polen (den Minus-Polen) der Gleichspannungsquellen 4a, 4b verbunden und über einen GND-Anschluss geerdet ist.

In einem nicht dargestellten Ausführungsbeispiel der Erfindung wurde 5 Gew.-% thermoplastisches Polyurethan (TPU, beschrieben in S. Baudis et al., Hard-block degradable thermoplastic urethane-elastomers for electrospun vascular prostheses, J. Polym. Sci. Part A Polym. Chem. 50 (2012) 1272-1280) in Hexafluorisopropanol gelöst und unter Einsatz einer erfindungsgemäßen Elektrospinnvorrichtung zu Endlos-Nanofasern verarbeitet, die auf der rotierenden Abscheidespindel zu einer Röhre aufgewickelt wurden. Der Abstand zwischen der Nadelspitze der Spinndüse und der Abscheidespindel betrug etwa 8 cm. Die Elektrospinnvorrichtung wurde als Ganzes in einen Faradayschen Käfig (Gehäuse) gestellt und in einem Reinraum der Klasse 1000 bei einer Temperatur von 24 °C und 34 % r. L. betrieben.
Der Zufluss der Polymerlösung in die Spinndüse wurde auf etwa 0,7 ml/h eingestellt und an der Spinndüse eine Spannung von etwa 12 kV angelegt. Die so auf der Abscheidespindel erhaltene elektrogesponnene Röhre wies einen Innendurchmesser von 1,5 mm und einer Wandstärke von etwa 300 µm auf.

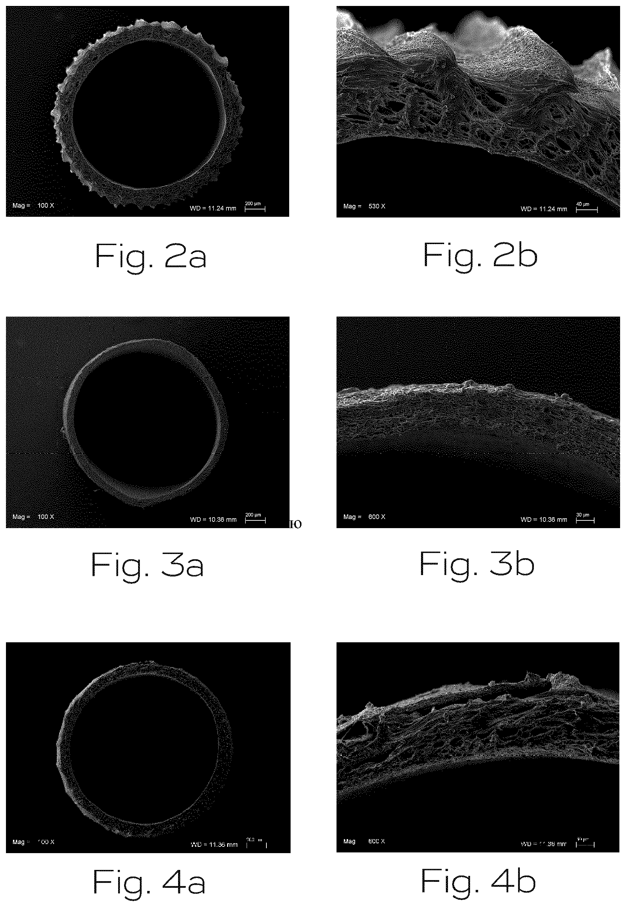

Figs. 2a - 3b zeigen mikroskopische Aufnahmen eines Querschnitts in Gesamtaufnahme und in vergrößerter Aufnahme verschiedener mit herkömmlichen Verfahren hergestellter Fasergerüste.

Bei dem Fasergerüst gemäß Figs. 2a - 2b wurde die Abscheidespindel 3 über die gesamte Dauer des Spinnvorgangs auf GND-Potential gehalten, entsprechend dem oben definierten Zustand (a). Es resultiert ein Fasergerüst mit zahlreichen radialen Wellen bzw. Lappen, die in Längsrichtung des gebildeten rohrförmigen Fasergerüsts verlaufen. Bei dem Fasergerüst gemäß Figs. 3a - 3b wurde die Abscheidespindel 3 entsprechend dem oben definierten Zustand (b) über die gesamte Dauer des Spinnvorgangs floatend, also ohne dediziertes Potential, gehalten, wobei eine Rückelektrode vorgesehen war, die während des Spinnvorgangs konstant auf GND-Potential gehalten wurde. Es resultiert ein Fasergerüst ohne Verwerfungen.

Figs. 4a - 4b zeigen mikroskopische Aufnahmen eines Querschnitts in Gesamtaufnahme und in vergrößerter Aufnahme verschiedener mit einem erfindungsgemäßen Verfahren hergestellter Fasergerüste. Bei dem Fasergerüst gemäß Figs. 4a - 4b wurde die Abscheidespindel 3 über eine Zeit t₁ während des Spinnvorgangs auf GND-Potential gehalten, und anschließend über eine Zeit t₂ floatend gehalten. Der Elektrospinnvorgang erstreckte sich dabei über den Zeitraum t₁ + t₂. Die Werte von t1 und t2 waren etwa 5 Minuten bzw. 15 Minuten; der gesamte Spinnvorgang etwa 20 Minuten. Es resultiert ein Fasergerüst mit zahlreichen Hohlräumen in der Außenwand, die in Längsrichtung des gebildeten rohrförmigen Fasergerüsts verlaufen, jedoch ohne äußere Verwerfungen. Ein derartiges Fasergerüst kann besonders vorteilhaft in der Gefäßchirurgie verwendet werden, da die Hohlräume unter anderem das Einwachsen von menschlichem Gewebe fördern. Die Erfindung beschränkt sich jedoch nicht auf die dargestellten Ausführungsbeispiele, sondern umfasst sämtliche Vorrichtungen und Verfahren im Rahmen der nachfolgenden Patentansprüche. Insbesondere beschränkt sich die Erfindung nicht auf Vorrichtungen und Verfahren zur Herstellung von Gefäßprothesen.

### Bezugszeichenliste

- 1: Reservoir
- 2: Spinndüse
- 3: Abscheidespindel
- 4a, 4b: Gleichspannungsquelle
- 5: Schalteinheit
- 6: Rückelektrode
- 7: Gehäuse
- 8: Materialstrahl
- 9: Achse
- 10: Fasergerüst
- 11: Steuereinheit

## Patentansprüche

1. Vorrichtung zur Herstellung eines anisotropen Fasergerüsts (10), insbesondere einer Gefäßprothese durch Elektrospinning, umfassend ein Reservoir (1), eine Spinndüse (2) zur Abgabe eines Materialstrahls (8) und eine relativ zur Spinndüse (2) rotierbare, elektrisch leitende Abscheidespindel (3), wobei zumindest eine vorzugsweise variable, zweipolige elektrische Gleichspannungsquelle (4, 4a, 4b) zur Bereitstellung einer elektrischen Potentialdifferenz zwischen der Spinndüse (2) und der Abscheidespindel (3) vorgesehen ist, und wobei ein die Abscheidespindel (3) und die Spinndüse (2) zumindest teilweise umschließendes, vorzugsweise geerdetes Gehäuse (7) vorgesehen ist,
**dadurch gekennzeichnet, dass**
eine gesteuerte Schalteinheit (5) vorgesehen ist, die dazu ausgebildet ist, während des Elektrospinnens die Abscheidespindel (3) zumindest einmal, vorzugsweise mehrmals von der Gleichspannungsquelle (4, 4a, 4b) zu trennen.

2. Vorrichtung nach Anspruch 1, **dadurch gekennzeichnet, dass** die Spinndüse (2) mit einem ersten elektrischen Pol der Gleichspannungsquelle (4) verbunden ist und die Abscheidespindel (3) mit einem zweiten elektrischen Pol der Gleichspannungsquelle (4) verbunden ist, wobei die Schalteinheit (5) dazu ausgebildet ist, während des Elektrospinnens den zweiten elektrischen Pol der Gleichspannungsquelle (4) zumindest einmal, vorzugsweise mehrmals von der Abscheidespindel (3) zu trennen.

3. Vorrichtung nach Anspruch 1, **dadurch gekennzeichnet, dass** zwei Gleichspannungsquellen (4a, 4b) vorgesehen sind, wobei die Spinndüse (2) mit einem ersten elektrischen Pol der **ersten Gleichspannungsquelle (4a)** verbunden ist und die Abscheidespindel (3) mit einem ersten elektrischen Pol der **zweiten Gleichspannungsquelle (4b)** verbunden ist, und wobei die Schalteinheit (5) dazu ausgebildet ist, während des Elektrospinnens den ersten elektrischen Pol der zweiten Gleichspannungsquelle (4b) zumindest einmal, vorzugsweise mehrmals von der Abscheidespindel (3) zu trennen.

4. Vorrichtung nach Anspruch 2 oder 3, **dadurch gekennzeichnet, dass** eine, bezüglich des ausgestoßenen Materialstrahls (8) hinter der Abscheidespindel (3) angeordnete **Rückelektrode** (6) vorgesehen ist, wobei die Schalteinheit (5) dazu ausgebildet ist, während des Elektrospinnens
a. den zweiten elektrischen Pol der Gleichspannungsquelle (4), oder
b. den ersten elektrischen Pol der zweiten Gleichspannungsquelle (4b) zumindest einmal, vorzugsweise mehrmals von der Abscheidespindel (3) zu trennen und mit der Rückelektrode (6) zu verbinden.

5. Vorrichtung nach einem der Ansprüche 2 bis 4, **dadurch gekennzeichnet, dass** das Gehäuse (7)
a. mit dem zweiten elektrischen Pol der Gleichspannungsquelle (4), oder
b. mit den zweiten elektrischen Polen der Gleichspannungsquellen (4a, 4b) verbunden ist.

6. Vorrichtung nach einem der Ansprüche 1 bis 5, **dadurch gekennzeichnet, dass** die Potentialdifferenz etwa 5 kV bis 20 kV, vorzugsweise 8 kV bis 17 kV, besonders bevorzugt 10 kV bis 15 kV beträgt, wobei das Potential an der Abscheidespindel (3) vorzugsweise kleiner ist als das Potential an der Spinndüse (2), insbesondere kleiner oder gleich 0 V, beispielsweise - 2kV.

7. Vorrichtung nach einem der Ansprüche 1 bis 6, **dadurch gekennzeichnet, dass** die Abscheidespindel (3) in einer im Wesentlichen orthogonal zum ausgestoßenen Materialstrahl (8) verlaufenden Achse (9) rotierbar und/oder entlang dieser Achse (9) verschiebbar ist, wobei die Abscheidespindel (3) insbesondere entlang der Achse (9) in einem sinusförmigen oder dreieckförmigen Schwingungsverlauf hin- und her-bewegbar ist.

8. Verfahren zur Herstellung eines anisotropen Fasergerüsts (10), insbesondere einer Gefäßprothese durch Elektrospinning, wobei ein Materialstrahl (8) aus einem Reservoir (1) über eine Spinndüse (2) auf eine rotierende, elektrisch leitende Abscheidespindel (3) geführt wird, wobei eine elektrische Potentialdifferenz zwischen der Spinndüse (2) und der Abscheidespindel (3) gebildet wird, indem die Spinndüse (2) und die Abscheidespindel (3) mit einer vorzugsweise variablen, zweipoligen elektrischen Gleichspannungsquelle (4, 4a, 4b) verbunden werden, **dadurch gekennzeichnet, dass**
eine gesteuerte Schalteinheit (5) während des Elektrospinnens die Abscheidespindel (3) zumindest einmal, vorzugsweise mehrmals von der Gleichspannungsquelle (4, 4a, 4b) trennt, sodass durch die Veränderung der elektrischen Potentialdifferenz zwischen der Abscheidespindel (3) und der Spinndüse (2) während des Elektrospinnens eine lokale Strukturierung des abgeschiedenen Fasergerüsts (10) erzielt wird.

9. Verfahren nach Anspruch 8, **dadurch gekennzeichnet, dass** die Potentialdifferenz gebildet wird, indem die Spinndüse (2) mit einem ersten elektrischen Pol der Gleichspannungsquelle (4) verbunden wird, und die Abscheidespindel (3) mit einem zweiten elektrischen Pol der Gleichspannungsquelle (4) verbunden wird, wobei die Schalteinheit (5) während des Elektrospinnens den zweiten elektrischen Pol der Gleichspannungsquelle (4) zumindest einmal, vorzugsweise mehrmals von der Abscheidespindel (3) trennt.

10. Verfahren nach Anspruch 9, **dadurch gekennzeichnet, dass** die Potentialdifferenz gebildet wird, indem die Spinndüse (2) mit einem ersten elektrischen Pol einer **ersten Gleichspannungsquelle** (4a) verbunden wird und die Abscheidespindel (3) mit einem ersten elektrischen Pol einer **zweiten Gleichspannungsquelle** (4b) verbunden wird, wobei die Schalteinheit (5) während des Elektrospinnens den ersten elektrischen Pol der zweiten Gleichspannungsquelle (4b) zumindest einmal, vorzugsweise mehrmals von der Abscheidespindel (3) trennt.

11. Verfahren nach Anspruch 9 oder 10, **dadurch gekennzeichnet, dass** die Schalteinheit (5) den zweiten elektrischen Pol der Gleichspannungsquelle (4), oder den ersten elektrischen Pol der zweiten Gleichspannungsquelle (4b) während des Elektrospinnens zumindest einmal, vorzugsweise mehrmals von der Abscheidespindel (3) trennt und mit einer, bezüglich des Materialstrahls (8) hinter der Abscheidespindel (3) angeordneten **Rückelektrode** (6) verbindet.

12. Verfahren nach einem der Ansprüche 8 bis 11, **dadurch gekennzeichnet, dass** die Abscheidespindel (3) während des Elektrospinnens relativ zur Spinndüse (2) entlang einer, in einer etwa orthogonal zum ausgestoßenen Materialstrahl (8) verlaufenden Achse (9) rotiert und/oder entlang dieser Achse (9) verschoben wird, insbesondere dass die Abscheidespindel (3) entlang der Achse (9) in einem sinusförmigen oder dreieckförmigen Schwingungsverlauf hin- und her-bewegt wird.

13. Verfahren nach einem der Ansprüche 8 bis 12, **dadurch gekennzeichnet, dass** die Schalteinheit (5) während des Elektrospinnens für eine erste Zeitdauer t₁ die Abscheidespindel (3) mit der Gleichspannungsquelle (4, 4a, 4b) verbindet, und anschließend für eine zweite Zeitdauer t₂ die Abscheidespindel (3) von der Gleichspannungsquelle (4, 4a, 4b) trennt, wobei die Zeitdauern t₁, t₂ jeweils etwa 5 bis 10 Minuten betragen und dieser Vorgang zur Herstellung des Fasergerüsts (10) etwa 5-10 mal wiederholt wird.

14. Verfahren nach einem der Ansprüche 8 bis 13, **dadurch gekennzeichnet, dass** die Förderrate des Materialstrahls (8) aus dem Reservoir (1) vorzugsweise etwa 0,5 - 2 ml/h, insbesondere etwa 0,7ml/h beträgt.

15. Verfahren nach einem der Ansprüche 8 bis 14, **dadurch gekennzeichnet, dass** der Materialstrahl (8) ein Polymer-Lösungsmittelgemisch umfasst, insbesondere
- eine Lösung aus 5 Gew.-% Polydioxanon (PDS) in Hexafluoro2propanol, oder
- eine Lösung aus 5 Gew.-% thermoplastisches Polyurethan (TPU) in Hexafluoro2propanol.
